# FASCICULE DE BREVET EUROPEEN

(11) **EP 1 229 868 B1**
(45) Date de publication et mention de la délivrance du brevet: **16.03.2005**
(21) Numéro de dépôt: 00981425.2
(22) Date de dépôt: 17.11.2000
(51) Int. Cl.: A61F 2/16

(54) **ANNEAU POUR SAC CAPSULAIRE ET ENSEMBLE CONSTITUE PAR UN TEL ANNEAU ET SON INJECTEUR**
KAPSELSACKRING UND SYSTEM BESTEHEND AUS RING UND DESSEN EINFÜHRUNGSVORRICHTUNG
RING FOR CAPSULAR BAG AND ASSEMBLY FORMED BY SAME AND ITS INJECTOR

(30) Priorité: 19.11.1999 FR 9914570
(43) Date de publication de la demande: 14.08.2002
(73) Titulaire: CORNEAL INDUSTRIE, 74370 Pringy (FR)
(72) Inventeur: PIETRINI, Pascal, F-44000 Nantes (FR); JEANNIN, Lionel, F-74330 Choisy (FR)
(74) Mandataire: Dronne, Guy
(86) Numéro de dépôt international: PCT/FR2000/003195
(87) Numéro de publication internationale: WO 2001/035868

(56) Documents cités:
- EP-A- 0 544 948
- EP-A- 0 884 031
- FR-A- 2 754 173

## Description

La présente invention a pour objet un anneau pour sac capsulaire et un ensemble constitué par un tel anneau et son injecteur.

De façon plus précise, l'invention concerne un anneau élastiquement déformable qui est destiné à être mis en place dans le sac capsulaire après l'ablation du cristallin en vue de maintenir la forme circulaire naturelle du sac capsulaire, et notamment mais non exclusivement, pour permettre ultérieurement une mise en place aisée d'un implant intraoculaire dans le sac capsulaire.

On sait que l'opération dite de la cataracte consiste à procéder à l'ablation du cristallin tout en conservant l'enveloppe du cristallin appelée sac capsulaire. Cependant, celui-ci étant constitué par une membrane très fine qui est reliée à la paroi interne de l'oeil par une couronne de fibrilles appelées zonules, cette membrane tend à ne pas conserver sa forme circulaire uniforme régulière.

On sait également qu'après l'ablation du cristallin, on procède à la mise en place dans le sac capsulaire d'un implant intraoculaire qui consiste essentiellement en une partie optique de forme sensiblement circulaire et en une partie haptique élastiquement déformable qui sert à centrer la partie optique de l'implant intraoculaire, de telle manière que son axe optique coïncide sensiblement avec l'axe optique de l'oeil. Pour que les parties haptiques remplissent convenablement leur fonction, c'est-à-dire qu'elles maintiennent le centrage de la partie optique de l'implant, il est nécessaire que la périphérie du sac capsulaire sur laquelle s'appuient les extrémités libres des parties haptiques conserve sa forme circulaire en évitant notamment la formation de plis dans le sac capsulaire, pour prévenir des complications telles que des déchirures zonulaires et des rétractions capsulaires.

Pour maintenir la forme circulaire du sac capsulaire, on a déjà proposé de mettre en place dans celui-ci un anneau élastique le plus souvent réalisé en PMMA dont l'expansion élastique maintient la forme circulaire du sac capsulaire. Subsidiairement, par la pression que l'anneau exerce sur la membrane constituant le sac capsulaire, il évite ou au moins limite la prolifération de cellules parasites qui pourraient venir obscurcir le sac capsulaire et donc altérer la vision du patient. De tels anneaux pour sac capsulaire sont notamment décrits dans la demande de brevet européen 884 031. L'anneau qui y est décrit a une forme symétrique. Cette forme ne permet pas d'éviter la formation de plis dans le sac capsulaire lors de la mise en place de l'anneau. En outre, cette forme ne permet pas d'obtenir une pression sensiblement identique sur toute la périphérie du sac capsulaire.

Un objet de la présente invention est de fournir un anneau pour sac capsulaire dont la forme facilite sa mise en place dans le sac capsulaire sans provoquer la formation de plis dans le sac capsulaire et permet une meilleure répartition et donc une meilleure efficacité de la pression exercée par l'anneau sur la périphérie du sac capsulaire afin d'améliorer encore le maintien de la forme naturelle circulaire de ce sac capsulaire.

Pour atteindre ce but selon l'invention, l'anneau pour sac capsulaire constitué par un élément allongé courbe présentant une première extrémité et une deuxième extrémité munie de moyens de manipulation, ledit élément allongé présentant un plan de symétrie, une épaisseur sensiblement constante selon la direction orthogonale audit plan de symétrie, et des bords externe et interne, ledit bord externe de l'élément allongé, au repos et dans son plan de symétrie, est disposé sensiblement tangentiellement en un point A à un cercle de centre O et de rayon R au moins égal au rayon du sac capsulaire, ledit bord externe étant disposé à l'extérieur dudit cercle ledit anneau se caractérisant en ce que ledit élément allongé comporte, en outre, une première branche s'étendant entre ladite première extrémité et un point B et une deuxième branche s'étendant entre ledit point B et ladite deuxième extrémité, ledit élément allongé ayant une longueur sensiblement au moins égale à celle du périmètre dudit cercle de centre O, le bord externe de ladite première branche étant défini par une portion de cercle de centre O', et de rayon R' (avec R < R' < R x 1,10) et en ce que le bord externe de la deuxième branche dudit élément allongé est défini par une portion de spirale hyperbolique de centre O et raccordée tangentiellement à ladite portion de cercle audit point B, proche dudit point A de tangence par quoi :
- la longueur de tout segment joignant le centre O à un point M' du bord externe de la deuxième branche est supérieure à la longueur du segment joignant le centre O du cercle à un point M' du bord externe de la première branche, le point M étant tel que l'angle que fait le segment OM avec le diamètre médian joignant le centre O au point de tangence A soit égal à l'angle que fait le segment OM' avec ledit diamètre médian ; et
- le rayon de courbure en tout point M' du bord externe de la deuxième branche est supérieur au rayon R'.

On comprend que l'anneau comporte une première branche qui a la forme sensiblement d'un arc de cercle dont le rayon est légèrement supérieur à celui du sac capsulaire et par une deuxième branche en forme de portion de spirale hyperbolique. La première branche est celle qui est introduite en premier dans le sac capsulaire. Du fait que cette branche correspond à peu près à un demi-cercle ayant un rayon proche de celui du sac capsulaire, la mise en place de cette première branche dans le sac capsulaire est aisée. Ce n'est que lorsque la deuxième branche en forme de spirale est introduite dans le sac capsulaire que l'anneau subit une déformation élastique significative de sa deuxième branche. De plus, en raison de sa forme en spirale hyperbolique, l'excès de diamètre de l'anneau dans cette deuxième branche par rapport au diamètre du sac capsulaire ne croît que progressivement. Cela facilite la mise en place de la deuxième branche de l'anneau et donc de l'anneau entier, puisque la compression de la deuxième branche est progressive.

En outre, la déformation élastique de l'anneau qui produit le maintien de la forme du sac capsulaire étant répartie le long de la deuxième branche de l'anneau qui correspond sensiblement à un demi-cercle, cela favorise la répartition des contraintes appliquées au sac capsulaire et donc évite les risques de formation de plis dans ledit sac.

Un autre problème qui se pose est celui de la façon d'introduire l'anneau à l'intérieur de l'oeil et du sac capsulaire. Pour cela, il est connu d'utiliser un injecteur qui se termine par une canule de diamètre réduit pour permettre son insertion dans l'oeil à travers une incision de dimension réduite, typiquement de l'ordre de 2,5 à 4 mm. L'anneau de sac capsulaire comporte au moins à l'une de ses extrémités un orifice dans lequel peut être engagé un crochet fixé à l'extrémité d'un fil qui permet l'introduction de l'ensemble de l'anneau dans la canule puis son éjection hors de la canule lorsque l'extrémité de celle-ci a été introduite dans l'oeil. La mise en place de l'extrémité de l'anneau sur le crochet de l'injecteur nécessite un certain nombre de manipulations relativement délicates en raison des faibles dimensions de cet anneau et du crochet à l'extrémité du fil de l'injecteur.

Il existe donc un réel problème relatif à la mise en place de l'anneau de sac capsulaire à l'intérieur de l'injecteur en vue de son insertion dans l'oeil.

Un autre objet de l'invention est de fournir un système d'anneau pour sac capsulaire associé à l'injecteur qui évite les manipulations préalables mentionnées ci-dessus.

Le système d'anneau pour sac capsulaire se caractérise en ce qu'il comprend :
- un anneau pour sac capsulaire comprenant deux branches dissymétriques présentant des rayons de courbure différents ; et
- un injecteur d'anneau pour mettre en place ledit anneau dans le sac capsulaire, ledit injecteur comprenant :
   - une canule munie d'un passage axial ;
   - un corps muni d'un mécanisme de manipulation de l'anneau, ledit mécanisme comportant un fil souple engagé dans ledit passage axial, ledit fil présentant une première extrémité munie d'un organe de manipulation apte à coopérer avec des moyens de manipulation de l'anneau prévus à une extrémité de celui-ci pour assurer la solidarisation temporaire dudit fil et dudit anneau et une deuxième extrémité solidaire de moyens de déplacement dudit fil dans ledit passage ;
et en ce que ledit organe de manipulation est solidaire desdits moyens de manipulation, ledit anneau est engagé dans le passage axial de ladite canule par l'extrémité de sa branche présentant le plus grand rayon de courbure sur une longueur convenable de sa deuxième branche sans qu'une contrainte mécanique soit appliquée à la branche.

On comprend que, grâce au fait que, selon l'invention, la branche de l'anneau introduite en partie dans la canule présente un rayon de courbure beaucoup plus grand que celui que l'on trouve dans les anneaux de l'art antérieur, il est possible d'introduire une extrémité de cette branche dans la canule de l'injecteur et de stocker cet ensemble jusqu'à son utilisation par un chirurgien.

En effet, grâce à ce rayon de courbure important, l'extrémité de la deuxième branche de l'anneau peut rester stockée dans la canule sans que celle-ci n'applique de déformation significative à la branche de l'anneau. Il faut souligner en effet qu'une telle disposition est impossible avec des anneaux de type standard puisque l'extrémité de ceux-ci présente une courbure importante qui induirait des contraintes dans cette extrémité de l'anneau susceptible d'en diminuer sensiblement la résistance mécanique au bout d'un certain temps de stockage.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles :
- la figure 1 est une vue de dessus d'un anneau pour sac capsulaire selon l'invention ;
- la figure 2 est une vue en coupe longitudinale d'un premier mode de réalisation d'un injecteur d'anneau ;
- les figures 3a et 3b sont des vues en perspective et en coupe partielle d'une partie de l'injecteur de la figure 2 permettant son verrouillage ;
- la figure 4 est une vue partielle en coupe longitudinale montrant la mise en place de l'anneau dans la canule de l'injecteur ;
- la figure 5 est une vue de détail de la figure 4 montrant la position de l'extrémité de l'anneau dans la canule ;
- la figure 6 montre un mode de réalisation de l'organe de solidarisation de l'anneau avec la canule.
- la figure 7 est une vue en coupe longitudinale d'un deuxième mode de réalisation de l'injecteur ;
- la figure 7a est une vue en coupe selon la ligne A-A de la figure 7 ; et
- la figure 7b est une vue partielle en perspective de l'injecteur de la figure 7.

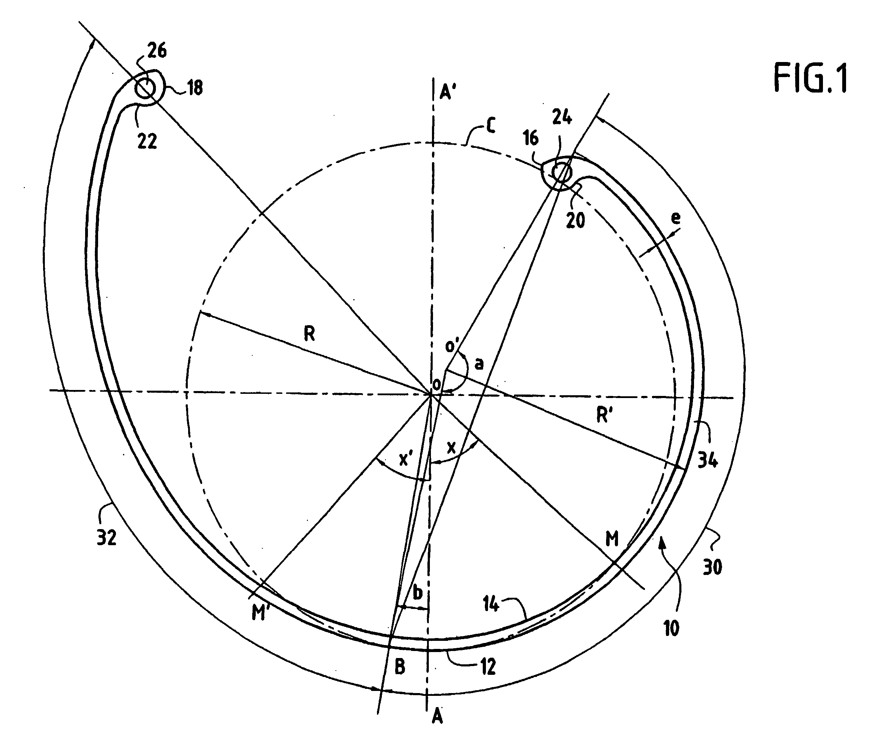

En se référant tout d'abord à la figure 1, on va décrire l'anneau de sac capsulaire selon l'invention dans sa forme au repos, c'est-à-dire lorsqu'aucune contrainte mécanique ne lui est appliquée. Celui-ci est réalisé de préférence en PMMA qui est un matériau biocompatible et qui est couramment utilisé pour la réalisation d'implants intraoculaires et en particulier pour les parties haptiques de ces implants.

L'anneau 10 peut être défini par référence à un cercle C de centre O et de rayon R, ce rayon étant sensiblement égal au rayon du sac capsulaire dans lequel l'anneau doit être mis en place. L'anneau 10 est tangent extérieurement au cercle C en un point A. Plus précisément, le cercle C est tangent au bord externe 12 de l'anneau 10, cet anneau comportant par ailleurs un bord interne 14. L'anneau se présente sous la forme d'une pièce allongée comportant une première extrémité 16 et une deuxième extrémité 18. Dans ce mode de réalisation, la largeur de l'anneau, c'est-à-dire la distance entre les bords 12 et 14, est constante. Par exemple, elle vaut 0,17 mm. L'épaisseur de l'anneau selon une direction perpendiculaire à son plan de symétrie est sensiblement constante. Par exemple, elle est égale à 0,17 mm. Seules les portions terminales 20 et 22 correspondant aux extrémités 16 et 18 présentent une largeur accrue pour permettre la réalisation de trous de manipulation de l'anneau 24 et 26. Par partie courante de l'anneau, on considère la totalité de la longueur de l'anneau à l'exception des parties terminales 20 et 22.

Si l'on revient à la définition de la forme de l'anneau, celui-ci comprend une première branche 30 qui s'étend depuis sa première extrémité 16 jusqu'à un point B du bord externe, le point B étant décalé par rapport au point de tangence A d'un angle au centre du cercle C qui est égal à b. De préférence, l'angle au centre b est compris entre 1° et 15° et le point B est situé d'un même côté que la deuxième branche par rapport au diamètre médian AA'. L'anneau comporte une deuxième branche 32 qui s'étend du point B à la deuxième extrémité de l'anneau 18.

La première branche 30 a la forme générale d'une portion de cercle 34 de centre O' et de rayon R', le centre O' n'étant pas en général confondu avec le centre O du cercle C. Le rayon R' est compris entre R et R + 10 % de R. Par rapport au centre O' de l'arc de cercle 34, l'angle au centre a sous lequel cet arc de cercle est vu depuis le point O' est inférieur à 180° et, de préférence, est supérieur à 120°.

On comprend que la première branche 30 de l'anneau a la forme générale d'un arc de cercle légèrement inférieur au demi-cercle et ayant un rayon R' légèrement supérieur au rayon du sac capsulaire.

En ce qui concerne le bord externe 12 de la deuxième branche 32 de l'anneau, il est défini par une spirale hyperbolique ou, plus précisément, par une portion de spirale hyperbolique de centre O. Dans le mode particulier de réalisation, l'équation de la portion de spirale en coordonnée polaire est r = 17/q, q étant l'angle au centre.

Du fait de la forme des branches 30 et 32 de l'anneau, on comprend que, si l'on considère un point M' du bord externe de la deuxième branche 32 qui fait un angle x avec le diamètre médian AA' du cercle C et le point M du bord externe de la première branche 30 faisant le même angle x avec le diamètre AA', la distance OM' est toujours supérieure à la distance OM. On comprend également que le rayon de courbure de la deuxième branche 32 est largement supérieur au rayon de courbure de la première branche, ce rayon de courbure allant en augmentant depuis le point B vers la deuxième extrémité 18 de l'anneau.

Pour les raisons qui viennent d'être exposées quant à la forme des deux branches de l'anneau, on comprend qu'il permet effectivement d'obtenir une mise en place aisée de cet anneau dans le sac capsulaire.

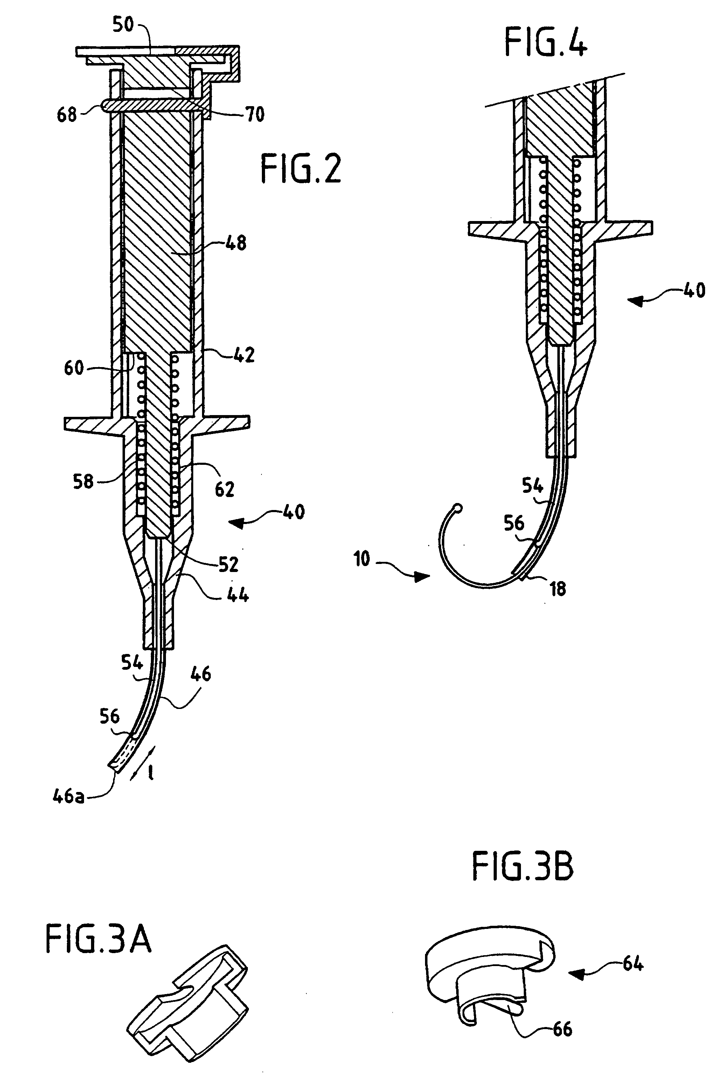

Sur la figure 2, on a représenté un injecteur d'anneau 40. Celui-ci comporte un corps cylindrique 42 se terminant par une portion conique 44 sur laquelle est fixée une canule en forme d'arc de cercle 46. A l'intérieur du corps 42 peut se déplacer un piston 48 qui comporte une tête d'actionnement manuel 50 et dont la deuxième extrémité 52 est solidaire de l'extrémité d'un fil métallique 54 qui peut se déplacer dans la canule 46 sous l'effet des déplacements du piston 48. Comme on l'expliquera plus en détail ultérieurement, le fil 54 se termine par un crochet 56 permettant la fixation de la deuxième extrémité 18 de l'anneau grâce à la présence du trou de manipulation 26. Un ressort de rappel 58 interposé entre un épaulement 60 du piston 48 et un épaulement 62 du corps de l'injecteur tend à faire entrer le fil 54 dans la canule 46. Pour des raisons que l'on exposera ultérieurement, de préférence l'injecteur 40 est équipé d'une pièce amovible formant un clip 64 qui comporte un ergot 66 qui peut être engagé dans des trous 68 du corps de l'injecteur et dans un alésage 70 du piston 48 de l'injecteur. Lorsque la pièce 64 est mise en place, le piston 48 et donc le crochet du fil 54 de l'injecteur sont immobilisés dans une position précise qui est en retrait d'une longueur I par rapport à l'ouverture 46a de la canule.

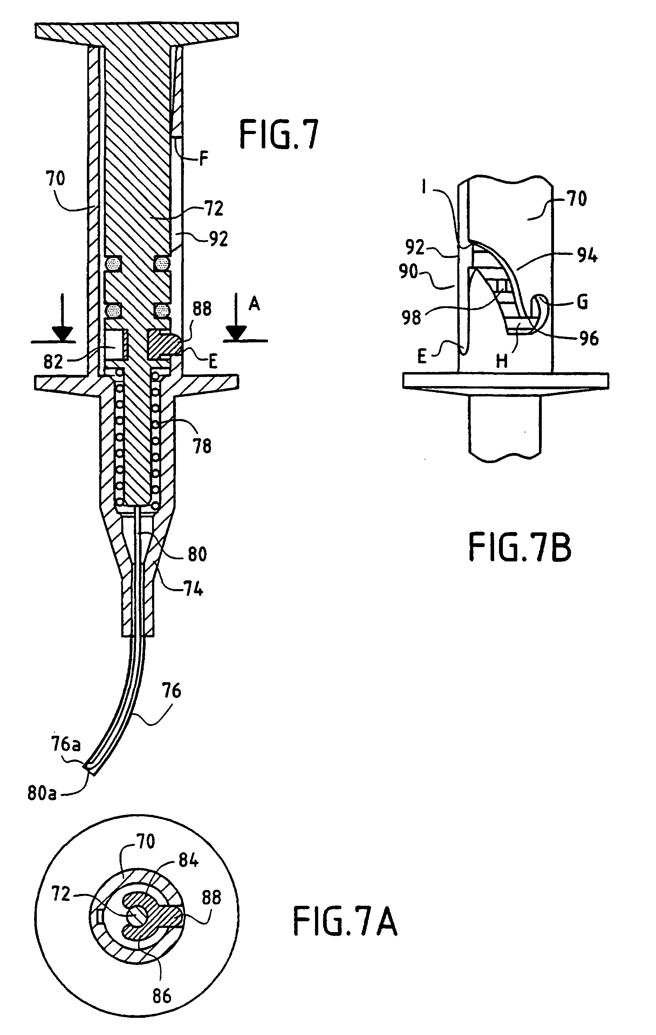

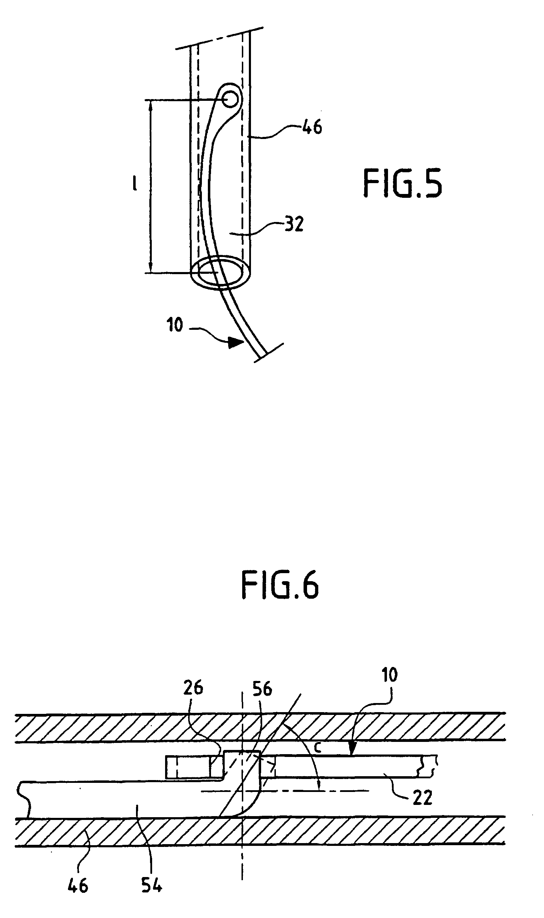

Sur la figure 4, on a représenté l'injecteur 40 équipé d'un anneau de sac capsulaire 10 dont la deuxième extrémité 18 pénètre dans l'extrémité de la canule et qui est solidaire du fil 54 par l'intermédiaire du crochet 56. Comme le montre mieux la figure 5, la deuxième extrémité de la deuxième branche 32 de l'anneau 10 pénètre dans la canule 46 d'une longueur I qui est telle que l'extrémité de l'anneau puisse être logée sans qu'aucune contrainte mécanique significative ne lui soit appliquée. Ainsi qu'on l'a déjà expliqué, ce résultat particulièrement favorable est obtenu grâce au fait que le rayon de courbure de la deuxième extrémité de la deuxième branche 32 de l'anneau est très supérieur à celui que l'on rencontre dans les anneaux pour sac capsulaire de l'état de la technique.

De préférence, la longueur I est de 4 à 5 mm.

La figure 6 montre plus en détails un mode préféré de réalisation du crochet 56 du fil 54 de l'injecteur. Il est constitué par une partie coudée de l'extrémité du fil. L'angle c que fait la partie coudée avec la partie courante du fil est compris entre 45° et 90°. On comprend que, grâce à la forme du crochet, celui-ci est maintenu dans l'évidement 26 de l'anneau tant que l'extrémité de l'anneau est à l'intérieur de la canule 46. Lorsque cette extrémité en est sortie, par un mouvement simple de l'injecteur, le chirurgien peut désolidariser l'anneau de l'injecteur.

Dans un mode préféré de réalisation, la canule a un évidement interne à section elliptique, dont les axes sont égaux à 1,1 mm et 0,6 mm. Le fil 54 a un diamètre de 0,3 mm et l'évidement 26 a un diamètre de 0,4 mm.

On comprend que, grâce à l'absence de contrainte mécanique appliquée à l'extrémité de l'anneau, il est possible de stocker pendant un temps important l'ensemble constitué par l'injecteur et par l'anneau 10 dont la deuxième extrémité est engagée dans la canule de l'injecteur et reliée au fil 54 de cet injecteur. L'ensemble est disposé dans une enveloppe présentant une étanchéité relative autorisant le passage du gaz (oxyde d'éthylène) utilisé pour stériliser l'ensemble constitué par l'injecteur et l'anneau.

Ainsi, lorsque le chirurgien veut mettre en place l'anneau dans le sac capsulaire d'un patient, l'anneau est déjà relié au fil 54 de l'injecteur. Il suffit au chirurgien de retirer la pièce formant clip 64 et, sous l'effet du ressort de rappel, le piston 48 se déplace vers l'arrière provoquant l'introduction de la totalité de l'anneau 10 à l'intérieur de la canule 46. Le chirurgien introduit alors l'extrémité 46a de la canule dans l'oeil du patient par l'incision qui a été préalablement réalisée et il lui suffit de repousser le piston pour faire sortir progressivement l'anneau, celui-ci pénétrant petit à petit dans le sac capsulaire en commençant par sa première branche dont on a déjà expliqué qu'elle a sensiblement la forme d'un demi-cercle correspondant sensiblement à la forme naturelle de la périphérie du sac capsulaire.

Sur les figures 7, 7a et 7b, on a représenté un deuxième mode de réalisation de l'injecteur. Celui-ci comprend un corps creux cylindrique 70 dans lequel peut se déplacer un piston 72. L'extrémité conique 74 du corps 70 est équipée d'une canule 76 identique à la canule 46 de la figure 2. Un ressort de rappel 78 tend à écarter le piston 72 de l'extrémité tronconique 74. Un fil d'accrochage 80 est solidaire de l'extrémité 72a et peut se déplacer dans la canule 76.

Le piston 72 comporte une gorge annulaire 82 dans laquelle est monté un organe de déplacement 84 constitué par une bague fendue 86, engagée dans la gorge 82, et un doigt de déplacement 88. Ainsi, le doigt 88 est solidaire en translation du piston 72, mais libre en rotation. Le doigt 88 pénètre dans une fente 90 ménagée dans le corps 72 de l'injecteur. La forme de la fente 90 est mieux visible sur la figure 7b. Cette fente comprend une partie rectiligne 92 parallèle à l'axe du corps de l'injecteur et une partie déviée 94. La partie rectiligne 92 s'étend entre un point E proche de l'extrémité tronconique et un point F (figure 7). Lorsque le doigt 88 occupe la position E, le fil 80 est entièrement sorti de la canule et l'anneau peut être décroché du fil. Lorsque le doigt 88 est dans la position F, l'anneau est entièrement rentré dans la canule 76. C'est la position initiale d'insertion.

La partie déviée 94 de la fente définit la position de stockage. La partie 94 de la fente comprend une première branche 96 s'étendant entre les points G et H, le point G correspondant à la position de stockage de l'anneau dans laquelle l'anneau n'est que très partiellement logé dans la canule comme on l'a déjà exposé. La deuxième branche 98 s'étend du point H à un point I de raccordement avec la fente rectiligne 92.

En position de stockage, le doigt 88 est maintenu de façon stable dans la positon G sous l'effet du ressort 78 en raison de la présence du coude constitué par le point H.

Lorsque le chirurgien veut se servir de l'injecteur, il doit exercer une pression sur la tête du piston 72 jusqu'à ce que le doigt 88 franchisse le coude H de la fente déviée 94. A partir de cette position, sous l'effet du ressort 78, le doigt 88 suit la portion de fente 96 puis la fente rectiligne 92 jusqu'au point F. L'anneau est alors entièrement engagé dans la canule. L'ensemble est prêt pour procéder à l'injection de l'anneau.

Il suffit alors au praticien d'exercer une pression sur la tête du piston 72 pour faire sortir progressivement l'anneau de la canule de l'injecteur, le doigt 88 se déplaçant dans la fente rectiligne 92.

Lorsque le doigt 88 arrive au point E, l'anneau est entièrement mis en place dans l'oeil du patient et cet anneau peut être décroché de l'extrémité du fil 80.

## Revendications

1. Anneau pour sac capsulaire constitué par un élément allongé courbe (10) présentant une première extrémité (16) et une deuxième extrémité (18) munie de moyens de manipulation, ledit élément allongé présentant un plan de symétrie, une épaisseur sensiblement constante selon la direction orthogonale audit plan de symétrie, et des bords externe (12) et interne (14), ledit bord externe (12) de l'élément allongé, au repos et dans son plan de symétrie, est disposé sensiblement tangentiellement en un point A à un cercle de centre O et de rayon R au moins égal au rayon du sac capsulaire, ledit bord externe étant disposé à l'extérieur dudit cercle, ledit anneau **se caractérisant en ce que** ledit élément allongé comporte, en outre, une première branche (30) s'étendant entre ladite première extrémité (16) et un point B et une deuxième branche (32) s'étendant entre ledit point B et ladite deuxième extrémité (18), ledit élément allongé ayant une longueur sensiblement au moins égale à celle du périmètre dudit cercle de centre O, le bord externe de ladite première branche étant défini par une portion de cercle de centre O', et de rayon R' (avec R < R' < R x 1,10) et **en ce que** le bord externe de la deuxième branche dudit élément allongé est défini par une portion de spirale hyperbolique de centre O et raccordée tangentiellement à ladite portion de cercle audit point B, par quoi :
- la longueur de tout segment de droite joignant le centre O à un point M' du bord externe de la deuxième branche est supérieure à la longueur du segment de droite joignant la centre O du cercle à un point M du bord externe de la première branche, le point M étant tel que l'angle que fait le segment de droite OM avec le diamètre médian joignant la centre O au point de tangence A soit égal à l'angle que fait le segment de droite OM' avec ledit diamètre médian ; et
- le rayon de courbure en tout point M' du bord externe de la deuxième branche est supérieur au rayon R'.

2. Anneau selon la revendication 1, **caractérisé en ce qu'**au moins ladite deuxième extrémité (18) de l'élément allongé présente dans le plan de symétrie une portion terminale (22) ayant une largeur accrue, ladite portion étant munie d'un orifice (26) de manipulation, formant lesdits moyens de manipulation.

3. Anneau selon l'une quelconque des revendications 1 et 2, **caractérisé en ce que** ledit point de raccordement B entre les deux portions de courbe est disposé par rapport au diamètre médian du même côté que la deuxième portion de courbe.

4. Anneau selon l'une quelconque des revendications 1 à 3, **caractérisé en ce que** ledit élément allongé (10) présente dans sa partie courante une largeur sensiblement constante entre ses bords interne (14) et externe (12).

5. Anneau selon l'une quelconque des revendications 1 à 3, **caractérisé en ce que** la première branche (30) de l'élément allongé présente dans sa partie courante une largeur e sensiblement constante entre ses bord interne et externe et **en ce que** la deuxième branche (32) de l'élément allongé présente dans sa partie courante une largeur entre ses bord interne et externe qui va en augmentant du point B jusqu'à proximité de la deuxième extrémité (18) de la largeur e à une largeur e' (e' > e).

6. Anneau selon l'une quelconque des revendications 1 à 5, **caractérisé en ce que** l'angle au centre (a) correspondant à la portion de cercle de centre O' est inférieur à 180 degrés et supérieur à 120 degrés.

7. Anneau selon l'une quelconque des revendications 1 à 6, **caractérisé en ce qu'**il est réalisé en PMMA.

8. Système d'anneau pour sac capsulaire, **caractérisé en ce qu'**il comprend :
- un anneau (10) pour sac capsulaire selon l'une quelconque des revendications 1 à 7 ; et
- un injecteur d'anneau (40) pour mettre en place ledit anneau dans le sac capsulaire, ledit injecteur comprenant :
- une canule (46, 76) munie d'un passage axial ;
- un corps (42, 70) muni d'un mécanisme de manipulation de l'anneau, ledit mécanisme comportant un fil souple (54, 80) engagé dans ledit passage axial, ledit fil présentant une première extrémité munie d'un organe de manipulation (56) apte à coopérer avec les moyens de manipulation (26) de l'anneau pour assurer la solidarisation temporaire dudit fil et dudit anneau et une deuxième extrémité solidaire de moyens de déplacement (48, 72) dudit fil dans ledit passage ;
et **en ce que**, lorsque ledit organe de manipulation est solidaire desdits moyens de manipulation, ledit anneau est engagé dans le passage axial de ladite canule par sa deuxième extrémité sur une longueur, convenable de sa deuxième branche sans qu'une contrainte mécanique soit appliquée à la branche.

9. Système d'anneau selon la revendication 8, **caractérisé en ce qu'**il comprend en outre un emballage dans lequel sont placés ledit injecteur et ledit anneau engagé dans ladite canule.

10. Système d'anneau selon la revendication 9, **caractérisé en ce que** l'emballage est réalisé en un matériau permettant la stérilisation à l'aide d'un gaz.

## Patentansprüche

1. Kapselsackring, bestehend aus einem langgestreckten krummen Element (10), das ein erstes Ende (16) und ein zweites mit Manipulationsmitteln versehenes Ende (18) aufweist, wobei das langgestreckte Element eine Symmetrieebene, eine in der zur Symmetrieebene orthogonalen Richtung im Wesentlichen konstante Dicke und einen äußeren (12) und einen inneren Rand (14) aufweist, wobei der äußere Rand (12) des langgestreckten Elements im Ruhezustand und in seiner Symmetrieebene im Wesentlichen tangential in einem Punkt A an einem Kreis um einen Mittelpunkt O und mit einem Radius R, der zumindest gleich dem Radius des Kapselsackrings ist, angeordnet ist, wobei der äußere Rand außen am Kreis angebracht ist, wobei der Ring **dadurch gekennzeichnet ist, dass** das langgestreckte Element zudem einen ersten Zweig (30), der sich zwischen dem ersten Ende (16) und einem Punkt B erstreckt, und einen zweiten Zweig (32), der sich zwischen dem Punkt B und dem zweiten Ende (18) erstreckt, aufweist, wobei das langgestreckte Element eine Länge hat, die im Wesentlichen gleich der des Umfangs des Kreises um den Mittelpunkt O ist, wobei der äußere Rand des ersten Zweigs durch einen Teil des Kreises um einen Mittelpunkt O' mit einem Radius R' (mit R < R' < R x 1,10) definiert ist, und dadurch, dass der äußere Rand des zweiten Zweigs des langgestreckten Elements durch einen Teil einer hyperbolischen Spirale um den Mittelpunkt O definiert ist und tangential an dem Teil des Kreises im Punkt B verbunden ist, wobei:
- die Länge jedes Abschnitts einer Gerade, die den Mittelpunkt O mit einem Punkt M' des äußeren Rands des zweiten Zweigs verbindet, größer ist als die Länge des Abschnitts der Gerade, die das Zentrum O des Kreises mit einem Punkt M des äußeren Rands des ersten Zweigs verbindet, wobei der Punkt M derartig ist, dass der Winkel, die der Abschnitt der Gerade OM mit dem Mediandurchmesser, welcher den Mittelpunkt O mit dem Berührungspunkt A verbindet, bildet, gleich dem Winkel ist, den der Abschnitt der Geraden OM' mit dem Mediandurchmesser bildet; und
- der Krümmungsradius in jedem Punkt M' des äußeren Rands des zweiten Zweigs größer ist als der Radius R'.

2. Ring gemäß Anspruch 1, **dadurch gekennzeichnet, dass** mindestens das zweite Ende (18) des langgestreckten Elements in der Symmetrieebene ein Endteil (22) mit einer vergrößerten Länge aufweist, wobei das Teil mit einer Manipulationsöffnung (26) ausgestattet ist und so die Manipulationsmittel gebildet werden.

3. Ring gemäß einem der Ansprüche 1 und 2, **dadurch gekennzeichnet, dass** der Anschlusspunkt B zwischen den zwei Krümmungsteilen bezüglich des Mediandurchmessers auf derselben Seite wie der zweite Krümmungsteil angeordnet ist.

4. Ring gemäß einem der Ansprüche 1 bis 3, **dadurch gekennzeichnet, dass** das langgestreckte Element (10) in seinem regulären Abschnitt eine im Wesentlichen konstante Länge zwischen seinem inneren (14) und äußeren Rand (12) aufweist.

5. Ring gemäß einem der Ansprüche 1 bis 3, **dadurch gekennzeichnet, dass** der erste Zweig (30) des langgestreckten Elements in seinem regulären Abschnitt eine Länge e aufweist, die zwischen seinem inneren und äußeren Rand im Wesentlichen konstant ist, und dadurch, dass der zweite Zweig (32) des langgestreckten Elements in seinem regulären Abschnitt eine Länge zwischen seinem inneren und äußeren Rand aufweist, die ansteigend vom Punkt B bis in die Nähe des zweiten Endes (18) der Länge e bis zu einer Länge e' (e' > e) verläuft.

6. Ring gemäß einem der Ansprüche 1 bis 5, **dadurch gekennzeichnet, dass** der Winkel im Mittelpunkt (a), der dem Teil des Kreis mit Mittelpunkt O' entspricht, kleiner als 180 Grad und größer als 120 Grad ist.

7. Ring gemäß einem der Ansprüche 1 bis 6, **dadurch gekennzeichnet, dass** er aus PMMA verwirklicht ist.

8. Kapselsackringsystem, **dadurch gekennzeichnet, dass** es umfasst:
- einen Kapselsackring (10) gemäß einem der Ansprüche 1 bis 7; und
- einen Ringinjektor (40) zum Einsetzen des Rings in den Kapselsack, wobei der Ringinjektor umfasst:
- eine Kanüle (46, 76), die mit einem axialen Durchgang versehen ist;
- einen Körper (42, 70), der mit einem Ringmanipulationsmechanismus ausgestattet ist, wobei der Mechanismus einen verformbaren Draht (54, 80) umfasst, der in den axialen Durchgang eingreift, wobei der Draht ein erstes Ende, das mit einem Manipulationselement (56) versehen ist, das in der Lage ist, mit den Manipulationsmitteln (26) des Rings zusammen zu wirken, um das vorübergehende Verbinden des Drahts und des Rings sicher zu stellen, und ein zweites Ende umfasst, das mit Mitteln (48, 72) zum Verschieben des Drahts in dem Durchgang fest verbunden ist;
und dadurch, dass der Ring, wenn das Manipulationselement mit den Manipulationsmitteln fest verbunden ist, durch sein zweites Ende auf einer angemessenen Länge seines zweiten Zweigs, ohne dass ein mechanischer Zwang auf den Zweig angewandt wird, in den axialen Durchgang der Kanüle eingreift.

9. Kapselsackringsystem gemäß Anspruch 8, **dadurch gekennzeichnet, dass** es zudem eine Verpackung umfasst, in welcher der Injektor und der in die Kanüle eingreifende Ring angeordnet sind.

10. Kapselsackringsystem gemäß Anspruch 9, **dadurch gekennzeichnet, dass** die Verpackung aus einem Material verwirklich ist, das die Sterilisation mit Hilfe eines Gases gestattet.

## Claims

1. A capsular bag ring constituted by a curved elongate element (10) presenting a first end (16) and a second end (18) provided with handling means, said elongate element presenting a plane of symmetry, a thickness that is substantially constant in a direction orthogonal to said plane of symmetry, and outer and inner edges (12, 14), said outer edge (12) of the elongate element when at rest and in its plane of symmetry being disposed substantially tangentially at a point A to a circle of center O and of radius R that is not less than the radius of the capsular bag, said outer edge being located at the outside of said circle, said ring being **characterised in that** said elongate element further comprises a first branch (30) extending between said first end (16) and a point B, and a second branch (32) extending between said point B and said second end (18), said elongate element being of a length that is substantially not less than the length of the perimeter of said circle of center O, the outer edge of said first branch being defined by a portion of a circle of center O' and of radius R', where R < R' < 1.10R, and **in that** the outer edge of the second branch of said elongate element is defined by a portion of a hyperbolic spiral of center O and joining tangentially with said portion of a circle at said point B, whereby:
- the length of any segment joining the center O to a point M' of the outer edge of the second branch is greater than the length of the segment joining the center O of the circle to a point M of the outer edge of the first branch, the point M being such that the angle between the segment OM and the median diameter joining the center O to the tangential point A is equal to the angle between the segment OM' and said median diameter; and
- the radius of curvature at any point M' of the outer edge of the second branch is greater than the radius R'.

2. A ring according to claim 1, **characterised in that** at least said second end (18) of the elongate element presents a terminal portion (22) in the plane of symmetry that is of increased width, said portion being provided with a handling hole (26) forming said handling means.

3. A ring according to claim 1 or claim 2, **characterised in that** said connection point B between the two curved portions is located on the same side of the median diameter as is the second curved portion.

4. A ring according to any one of claims 1 to 3, **characterised in that** said elongate element (10) is of substantially constant width between its inner and outer edges (14, 12) in its main portion.

5. A ring according to any one of claims 1 to 3, **characterised in that** the first branch (30) of the elongate element presents a substantially constant width e in its main portion between its inner and outer edges, and **in that** the second branch (32) of the elongate element presents a width between its inner and outer edges in its main portion that increases going away from the point B to the vicinity of the second end (18) from the width e until a width e', where e' > e.

6. A ring according to any one of claims 1 to 5, **characterised in that** the angle at the centre (a) corresponding to the portion of a circle of centre O' is less than 180° and greater than 120°.

7. A ring according to any one of claims 1 to 6, **characterised in that** it is made of PMMA.

8. A capsular bag ring system, **characterised in that** it comprises:
- a capsular bag ring (10) according to any one of claims 1 to 7; and
- a ring injector (40) for putting said ring into place in the capsular bag, said injector comprising:
- a cannula (46, 76) provided with an axial passage;
- a body (42, 70) provided with a ring-handling mechanism, said mechanism comprising a flexible wire (54, 80) engaged in said axial passage, said wire having a first end provided with a handling member (56) suitable for co-operating with the handling means (26) of the ring to secure said wire temporarily to said ring, and a second end secured to means (48, 72) for moving said wire in said passage; and
**in that** when said handling member is secured to said handling means, said ring is engaged in the axial passage of said cannula via its second end over a suitable length of its second branch without applying any mechanical stress to the branch.

9. A ring system according to claim 8, **characterised in that** it further comprises a package containing said injector and said ring engaged in said cannula.

10. A ring system according to claim 9, **characterised in that** the package is made of a material that enables sterilization to be performed by means of a gas.
